**Europäisches Patentamt**

**European Patent Office**

**Office européen des brevets**

(19)

(11) Publication number: **0 070 167**

**A2**

(12) # EUROPEAN PATENT APPLICATION

(21) Application number: **82303614.0**

(22) Date of filing: **09.07.82**

(51) Int. Cl.³: **A 61 K 39/00**
**A 61 K 39/395**

(30) Priority: **14.07.81 US 283241**

(43) Date of publication of application:
**19.01.83 Bulletin 83/3**

(84) Designated Contracting States:
**BE CH DE FR GB IT LI NL SE**

(71) Applicant: **Nardi, Ronald V.**
**11 Crestview Drive**
**Willingboro New Jersey 08046(US)**

(71) Applicant: **Fernandes, Prabhavathi B.**
**501 Broadacres Road**
**Penn Valley Pennsylvania 18072(US)**

(72) Inventor: **Nardi, Ronald V.**
**11 Crestview Drive**
**Willingboro New Jersey 08046(US)**

(72) Inventor: **Fernandes, Prabhavathi B.**
**501 Broadacres Road**
**Penn Valley Pennsylvania 18072(US)**

(74) Representative: **Oliver, Roy Edward et al,**
**POLLAK,MERCER & TENCH High Holborn House 52-54**
**High Holborn**
**London WC1V 6RY(GB)**

(54) **Hypertension treatment.**

(57) A composition for treating mammalian hypertension, evidenced by the presence in a body fluid of the mammal of a protein associated with hypertension, comprises an agent which blocks the hypertensive effect of such a protein and contains an antibody to the protein.

For treating mammalian hypertension, the composition can comprise an antigen which is a protein associated with hypertension in a mammal.

Processes for treating hypertension in a mammal which has or is predisposed to hypertension, evidenced by the presence in a body fluid of the mammal of a protein associated with hypertension, essentially comprise administering to the mammal a dose of the composition comprising such an agent.

The agent can be made by immunizing a mammal by injecting it with a dose of an agent, such as a protein associated with hypertension and isolated from a body fluid of a mammal having or predisposed to hypertension, so as to induce in the mammal an antibody to the protein associated with hypertension, removing blood from the mammal when the antibody to the protein has formed and separating the antibody from the blood.

- 1 -

HYPERTENSION TREATMENT

DESCRIPTION

This invention relates to a new means for treating hypertension in a mammal, and more particularly in a human. By use of the diagnostic process described and claimed in our copending European Patent Application No 81301164.0 (Publication No. 0036336) and also referred to below, it is possible to determine in humans the existence of or predisposition to essential hypertension as opposed to secondary hypertension. The invention includes as a preferred embodiment an electrophoretic process for detecting, isolating and separating a specific protein or proteins associated with mammalian hypertension in body fluids which were previously undetected and unidentified. Hypertension is managed and treated by administering an effective dosage of an agent that blocks the hypertensive effect of the protein associated with hypertension. Active immunization of a mammal being treated with a protein associated with hypertension also reduces hypertension.

Hypertension is excessive blood pressure in the arterial system which, if left untreated, leads to disability and premature death. Hypertension is generally divided into two broad categories, essential hypertension and secondary hypertension.  Essential hypertension is a
familial or genetic form of elevation of blood pressure of
unknown cause.  Secondary hypertension is hypertension of
known organic origin such as that associated with renovascular or renal parenchymal disease. The management and
treatment for essential hypertension differs from that
used for secondary hypertension.

By the diagnostic process disclosed herein, it will
be possible to differentiate whether a patient who has
hypertension is suffering from essential hypertension or
from secondary hypertension.  Further, it will be.possible
to determine which patients may be predisposed to essential hypertension, although they presently may not have
elevated blood pressure.

The inventors' research indicates that the protein
associated with hypertension in mammals generally, and
with essential hypertension in humans, functions in some
way to be at least partly responsible for the hypertensive
effect.  Based on their research, the inventors believe
that patients with essential hypertension will respond to
a therapeutic approach that blocks the function of the
protein associated with hypertension.  As set forth hereinafter, there are several categories within this broad
therapeutic approach that are believed to be effective in
managing and treating hypertension.

This invention has primary use in the clinical
management of human patients.  However, the treatment of
other mammals, such as pets and livestock, is also considered to be within the scope of this invention.

Hypertension is a disease of epidemic proportion
affecting some 60 million people in the United States.
Hypertension is a major risk factor in the eventual development of significant atherosclerotic complications,
namely, myocardial infarction and stroke.  Accordingly,
it is very important to be able to diagnose and properly
treat hypertension, whether it is essential hypertension or secondary hypertension. In addition, hypertension costs the United States more than 8 billion dollars a year in medical costs, lost productivity and lost wages. A significant amount is spent on cost-ineffective investigations directed to exclude secondary hypertension associated with a variety of causes. However, it is only by exclusion of evident causes that diagnosis of essential hypertension can be made. This diagnostic process described herein is a definite and cost-effective process for the detection of a biochemical marker or markers of essential hypertension.

The dividing line based on blood pressure measurements between normotension (normal blood pressure) and hypertension is not clear. While certain guidelines have been proposed, there is no absolute blood pressure above which it can be said that high blood pressure or hypertension exists. This is important in that patients or other mammals who are considered normotensive may in fact be hypertensive, and vice versa. Thus, some patients should be treated for hypertension and others should not when the same blood pressure is exhibited. The detection technique will help alleviate the area of uncertainty between normotension and mild hypertension. Moreover, even when a patient undoubtedly has hypertension, there is frequently no convenient or certain method for determining whether the hypertension is the disease (essential hypertension) or whether it is caused by another disease (secondary hypertension). Since the managements and treatments differ, it is important to know which type of hypertension a patient has. The detection technique disclosed herein allows this determination.

The therapeutic treatment and management of hypertension according to the present invention is based upon the detection, isolation and separation of a protein associated with hypertension in mammals and a protein associated with essential hypertension in humans. The preferred process for detecting, isolating and separating the protein associated with hypertension is the use of high resolution discontinuous sodium dodecyl sulfate (sodium dodecyl sulfate will be abbreviated "SDS" hereinafter) polyacrylamide gel electrophoresis, although other qualitative and quantitative methods for the detection, isolation and separation of proteins in body fluids may be used.

SDS polyacrylamide gel electrophoresis is a technique that has been used in analyzing protein components of eukaryotic and prokaryotic preparations. For example, see Laemmli, U.K., Nature 227: 680-685, 1970; Ames, G.F.L., Journal of Biological Chemistry 249: 634-644, 1974; Weingarten, M.D., Lockwood, A.H., Hwo, S.Y. & Kirshner, M.W., Proceedings of the National Academy of Sciences, U.S.A. 72: 1858-1863, 1975; Sloboda, R.D., Dentler, W.C. & Rosenbaum, J.L., Biochemistry 15: 4497-4505, 1976; and Fernandes, P.B., Nardi, R.V. & Franklin, S.G., Analytical Biochemistry 91: 101-114, 1978.

Despite these investigations, it is believed that prior to the present invention no one has considered using an electrophoretic gel process for determining the differences between normotensive and hypertensive mammals, the differences between mammals having essential hypertension and mammals having secondary hypertension to detect, isolate and separate protein associated with hypertension for use in making antiserum to the protein and thereby manage and treat the hypertension. It is believed that this is partly because it is generally accepted that essential hypertension represents high blood pressure without evident cause, so that a specific search for biochemical "markers of disease", such as the proteins detected and identified herein, did not appear relevant. Furthermore, until the inventors discovered the existence of proteins associated with at least a predisposition to hypertension, no one could have conceived of a method of detecting and/or identifying them or any way to treat and manage hypertension based on them.

Electrophoretic gel analysis has been used to detect other diseases or pathological problems (see, for example, U.S. Patent 3,607,695 of Schneider, issued September 21, 1971 and U.S. Patent 3,687,833 of Parcells et al, issued August 29, 1972), but the use of gel electrophoresis has not been considered with respect to the determination of factors affecting hypertension.

## Summary Of The Invention

The present invention is based upon the discovery that a particular protein (or proteins) in body fluids is associated with hypertension in mammals, including human patients. The inventors have discovered that the protein or proteins are biochemical markers for humans with or predisposed to essential hypertension, as compared to secondary hypertension.

Because of an unusual migration characteristic noted in the detection of the protein or proteins associated with hypertension or with essential hypertension, as discussed in more detail hereinafter, it is not certain that there is only one protein associated with hypertension or with essential hypertension. For the sake of clarity, the singular word "protein" will be used throughout this application, rather than the term "protein or proteins" when discussing the protein or proteins associated with hypertension or with essential hypertension.

The present invention comprises a composition for treating mammalian hypertension evidenced by the presence in a body fluid of the mammal of a protein associated with hypertension, the composition comprising an agent which blocks the hypertensive effect of the protein associated with hypertension.

The present invention also comprises a process for treating hypertension in a mammal having or being predisposed to hypertension, evidenced by the presence in a body fluid of the mammal of a protein associated with hypertension, comprising administering to the mammal a dosage of an agent effective to block the hypertensive effect of the protein associated with hypertension.

The presently preferred agent contains antibody to the protein associated with hypertension.

Another aspect of the present invention includes a composition for treating mammalian hypertension comprising an antigen, the antigen being a protein associated with hypertension in a mammal.

Also included in the present invention is a process for treating mammalian hypertension in a mammal having or being predisposed to hypertension comprising immunizing the mammal by injecting a dosage of an agent effective to induce in the mammal antibody to a protein associated with hypertension in the mammal.

Brief Description Of The Drawings

Photographs and ink drawings are provided for the purpose of illustrating a preferred analytical technique for detecting, isolating and separating the protein associated with hypertension.  It should be understood, however, that this invention is not limited to the precise techniques, determinations and results illustrated in the photographs and drawings.

Figure 1 is a photograph of a SDS polyacrylamide gel comparing protein bands representative of proteins present in blood plasma from a spontaneously hypertensive rat with protein bands representative of proteins present in blood plasma from a normotensive control rat.

Figure 2 is an enlargement of the lower portion of Figure 1 which is of primary interest.

Figure 3 is a scan of the photograph of Figure 1 using a soft laser scanning densitometer in a high resolution mode.

Figure 4 is a photograph of a SDS polyacrylamide gel comparing protein bands representative of proteins in urine from a spontaneously hypertensive rat with protein bands representative of proteins present in urine from a normotensive control rat.

Figure 5 is photograph of a SDS polyacrylamide gel comparing protein bands representative of proteins in urine from a rat made hypertensive by a surgical technique to serve as a model of secondary renal hypertension with protein bands representative of proteins present in urine from a normotensive control rat subjected to a sham surgical operation.

Figure 6 is a photograph of a SDS polyacrylamide gel comparing protein bands representative of proteins in blood plasma of rats made hypertensive by a surgical technique to serve as models of secondary renal hypertension with protein bands representative of proteins in blood plasma from a normotensive control rat, and from a hypertensive rat rendered normotensive by removal of the ischemic kidney.

Figure 7 is an enlargement of the lower portion of Figure 6 which is of primary interest.

Figure 8 is a scan of the photograph of Figure 6 using a soft laser scanning densitometer in a high resolution mode.

Figure 9 is a photograph of a SDS polyacrylamide gel comparing protein bands representative of proteins in blood plasma from humans with essential hypertension with protein bands representative of proteins present in blood plasma from humans who are normotensive and from humans with secondary hypertension.

Figure 10 is a photograph of a SDS polyacrylamide gel having a uniform concentration of polyacrylamide comparing protein bands representative of proteins present in blood plasma from humans with essential hypertension with the protein bands representative of proteins present in blood plasma from normotensive humans and from humans with secondary hypertension.

Figure 11 is a photograph of a SDS polyacrylamide gel using a horizontal polyacrylamide gel concentration gradient showing protein bands representative of the migration of proteins present in blood plasma of a normotensive human.

Figure 12 is a photograph of a SDS polyacrylamide gel using a horizontal polyacrylamide gel concentration gradient showing protein bands representative of the migration of proteins present in blood plasma from a human with essential hypertension.

Description Of The Preferred Embodiments

The present invention will be described with respect to a particular process for diagnosing the presence of hypertension or a predisposition to hypertension in a mammal, diagnosing the presence of or predisposition to essential hypertension in humans, and for treating such hypertension and essential hypertension.

Since the therapeutic aspects of the present invention are based upon the detection of the protein associated with hypertension, the invention will be described and explained in several steps. First the detection of the protein will be described. The isolation of the protein associated with hypertension and preparation of immune serum containing antibody to the protein associated with hypertension will be described next. Then the therapeutic administration of the agents to reduce and thereby treat and manage hypertension will be described.

The particular process described in detail herein for detecting, isolating and separating the protein associated with hypertension is based on the process of discontinuous SDS polyacrylamide gel electrophoresis. However, it should be understood that the process for detecting the presence of the particular protein associated with hypertension and isolating and separating this protein from others in the body fluid containing them need not be limited to SDS polyacrylamide gel electrophoresis, electrophoresis techniques in general, or any other particular process or technique.  Thus, the protein associated with hypertension, once identified, will be a "marker" for the disease.  Anyone who detects, identifies, isolates or separates the marker protein by any analytical or preparative technique, be it qualitative or quantitative, will be using this invention.

SDS polyacrylamide gel electrophoresis has been chosen as the presently preferred process for detecting, isolating and separating the protein associated with hypertension because it is a readily available technique.  The equipment and reagents used in SDS polyacrylamide gel electrophoresis are readily available commercially and presently form part of the standard equipment of many clinical laboratories.  Additionally, a large number of samples of body fluids can be run simultaneously, efficiently and economically using standard SDS polyacrylamide gel electrophoresis equipment.  Other suitable methods of detecting and identifying the presence of a protein or proteins associated with hypertension may be the well-known analytical techniques including, for example and not by way of limitation, various chromatographic techniques, such as high pressure liquid chromatography, thin layer chromatography, starch gel chromatography, silica gel chromatography; other types of electrophoresis, such as starch gel electrophoresis, silica gel electrophoresis; antibody-antigen interactions and related immunological technology such as immune precipitation, immune electrophoresis, enzyme-linked immunosorbent assay and radioimmmunoassay, and the like.  Several of the electrophoretic and chromatographic techniques which are suitable for use in detecting the protein or proteins associated with hypertension are described in Smith, I., Ed., Chromatographic and Electrophoretic Techniques, Volume II, Zone Electrophoresis, 4th Edition, Year Book Medical Publishers, Inc., Chicago, 1976; Chapter 12, Payne, J. W., "Electrophoresis of Proteins on Sodium Dodecyl Sulphate Polyacrylamide Gels" generally describes the preferred process used with the present invention.

A small sample of the body fluid to be tested is applied to a solid electrophoretic support medium, preferably SDS polyacrylamide gel. It will be understood, however, that other support media may be used, such as for example cellulose acetate, cellulose nitrate, agar, agarose, paper, cellulose, silica gel, starch gel, and the like.

The apparatus used for discontinuous SDS polyacrylamide gel electrophoresis is widely available commercially, such as from Aquebogue Machine & Repair Shop, Aquebogue, N.Y. It generally comprises two glass plates separated from each other by spacer strips of inert material, such as methyl methylacrylate. The spacer strips are used between the plates along each side edge and the bottom edge. The assembly is clamped together and the edges are sealed such as by dripping agar or the like around the outside edges.

A resolving gel is poured between the plates until the space between the plates is approximately 75% to 90% full. After the gel has set, an inert spacer in the shape of a comb is inserted between the plates at the upper portion thereof to form sample wells to receive samples of the body fluid to be tested. A spacer gel is poured between the plates around the comb and allowed to set. The comb and the bottom spacer strip are then removed.

The sample receiving apparatus is then clamped in an electrophoresis apparatus so that an upper chamber of electrode buffer is in contact with the gel at the upper portion of the sample receiving apparatus and a lower electrode buffer is in contact with the gel at the lower portion of the sample receiving apparatus. A cathode is immersed in or connected to the upper buffer container and an anode is immersed in or connected to the lower buffer container.  Either constant current or constant voltage may be applied to the anode and cathode to cause the migration of proteins in the body fluid sample through the gel.  The use of constant current is presently preferred.  The amount of the current or voltage is well known to those of ordinary skill in the art. Typical currents are 30 milliamperes which is used for a run lasting about 4 to 4.5 hours.  8 milliamperes can be used for a run lasting about 16 to about 17 hours.  Typical voltages are 30-300 volts.  The current or voltage is maintained until the deired degree of migration of the proteins in the sample is achieved.

The general technique for preparing the resolving gel, spacer gel, electrode buffer and sample buffer are well known to those of ordinary skill in the art, having been described in Laemmli, U.K., Nature 227: 680-685, 1970; and Fernandes P. B., Nardi, R. V. and Franklin, S. G., Analytical Biochemistry 91: 101-114, 1978.

An example of a suitable resolving gel includes the following ingredients.  An acrylamide-bis-acrylamide stock solution is prepared using 60 g acrylamide and 1.6 g N,N'-methylene-bisacrylamide per 100 ml of solution, the balance being water.  Other formulations are possible. For example, other cross-linking agents may be used instead of N,N'-methylene-bis-acrylamide, such as, for example, dihydroxyethylene-bis-acrylamide or bis-acrylylcystamine.  As the crosslinking agent and the ratio of cross-linker to the monomer is changed, different characteristics result which can be tailored as desired by agent, such as acrylamide to N,N'-methylene-bis-acrylamide determines the sieving property of the gel and thereby the optimum resolution conditions.

The polymerization of the acrylamide and N,N'-methylenebis-acrylamide solution is catalyzed by N, N, N', N'-tetramethylene diamine (hereinafter "TEMED") and ammonium persulfate (hereinafter "APS"). Riboflavin and light may also be used to catalyze polymerization of the acrylamide gel. The resulting gel also contains Tris-HCl buffer with pH 8.8, SDS, and glycerol, if desired for purposes discussed hereinafter.

As used herein, the term "%" or "percent" means the weight to volume percent of the particular ingredient in the composition or component being described, unless otherwise indicated in the context of the description. Thus, for example, the following description is directed to the percentage of the ingredients in the resolving gel and the percentage of each is the final percentage of the ingredient in the resolving gel.

The Tris-HCl may be adjusted to yield a final concentration of about 0.15M to about 0.75M in the gel. The presently preferred concentration is 0.375M. The SDS concentration may be adjusted to a final concentration in the gel of up to about 0.5%, 0.1% being presently preferred. The acrylamide portion of the gel (and hence, the polyacrylamide gel) may be adjusted to have a uniform acrylamide concentration or a gradient of acrylamide concentration. When a uniform acrylamide concentration is used, it should be present in about 10% to about 20% to detect the specific variation in protein composition in samples of body fluids from mammals. The presently preferred uniform concentration of acrylamide is about 13% to about 14%. Uniform concentrations of acrylamide above 20% are possible, but do not appear to be more advantageous.

Gradients of acrylamide concentration may be used in the resolving gel with various end concentrations between about 5% and about 30% to detect specific variation in protein compositions in samples of body fluids from mammals being tested. Gradients of acrylamide concentration may be adjusted exponentially or linearly for example. Examples of satisfactory gradient concentrations of acrylamide include exponential gradients of about 8% to about 25%, about 12% to about 20%, and about 12% to about 30%; and linear gradients of about 10% to about 25%. Glycerol is not necessary but may be added to the acrylamide to stabilize the gradient concentrations when gradient concentrations are being used. The glycerol may be used in amounts of up to about 10%.

The spacer gel includes Tris-HCl buffer with pH 6.8, SDS, and acrylamide prepared from the stock solution of acrylamide and bis-acrylamide. The polymerization of the acrylamide solution is catalyzed with TEMED and APS. The acrylamide concentration may vary between about 3% and about 6%, 6% being the presently preferred concentration, since above 6% higher molecular weight proteins are sieved. The concentration of the Tris-HCl buffer may be adjusted to be about 0.060M to about 0.250M, 0.125M being presently preferred. The SDS concentration is the same as in the resolving gel.

An electrode buffer solution is poured into the upper and lower buffer chambers of the electrophoresis apparatus. The electrode buffer solution is prepared by adding 3.0 g of Tris, 14.4 g. glycine, SDS to a final concentration in the electrode buffer solution of 0.1%, and a sufficient amount of water to bring the total volume of the electrode buffer solution to 1 liter. The electrode buffer solution has an approximate pH 8.3. The concentrations of Tris and glycine may be doubled to change the time it takes for the proteins to migrate.

Body fluids to be tested for the presence of at least one protein associated with hypertension may include urine, blood plasma, blood serum or any other protein-containing body fluid. A sample of a body fluid to be tested, diluted if desired with deionized water, is mixed with an equal volume of sample buffer to give a sample solution. One composition for a suitable sample buffer includes about 0.050M to about 0.125M Tris-HCl pH 6.8, a preferred amount being 0.050M; about 5.0% to about 20% of glycerol, a preferred amount being 10%; about 1.0% to about 8% of SDS, a preferred amount being 4%. Prior to boiling the sample solution, a tracking dye, such as bromophenol blue, is used in the sample buffer. Other suitable dyes may be used instead of bromophenol blue. A reducing agent, such as 2-mercaptoethanol (having a final concentration in the total sample solution of about 5% to about 10%) is added. Other reducing agents such as dithiothreitol, dithioerythritol, etc., may be used to reduce the disulfide bonds in the amino acid groups in the proteins.

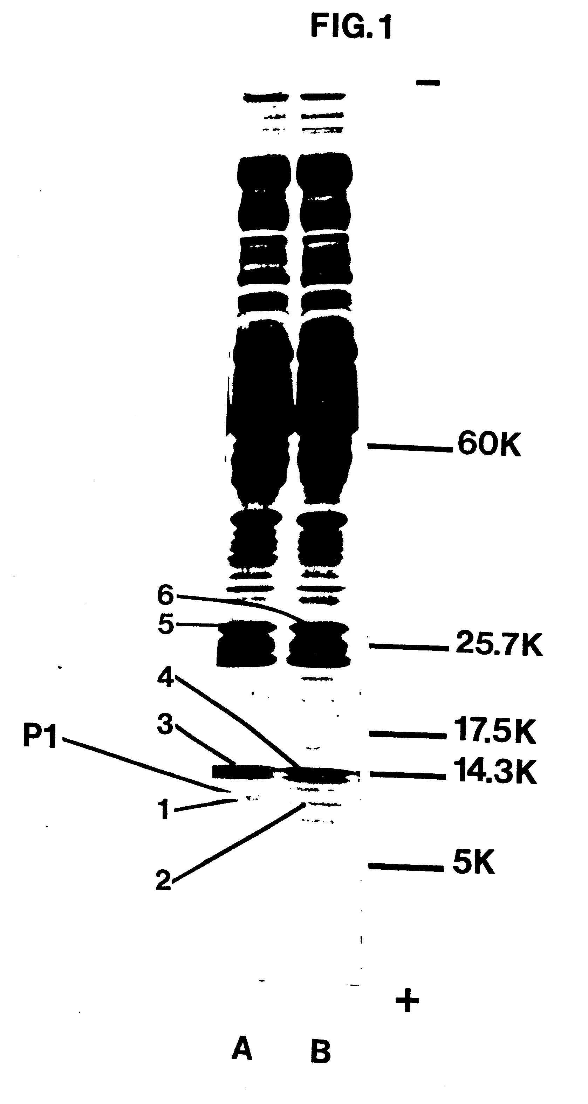

The sample solution is boiled for about 2 to about 5 minutes in a capped tube. After cooling, an aliquot of each of the various sample solutions are placed in each of the sample wells in the spacer gel, the current is applied and electrophoresis is performed. When the tracking dye and the proteins have migrated an appropriate distance, the current is shut off and visualization of the protein bands as illustrated in Figure 1, for example, is produced by standard Coomassie Brilliant Blue R 250 staining procedures. The gels may then be analyzed and dried. The gels may be photographed for recordation and/or analysis.

Detection Of Protein Associated With Hypertension

In a preferred embodiment, the protein associated with hypertension is detected by analytical, rather than preparative, discontinuous SDS polyacrylamide gel electrophoresis. A further preferred technique is to use a gradient of concentrations of polyacrylamide gel as the resolving gel in the electrophoretic techinque.

The detection of the protein associated with hypertension will now be described in more detail with reference to the following specific, non-limiting examples relating to the procedure, results and analysis of exemplary body fluids, namely urine and blood plasma, from laboratory rats and human patients.

## Example 1

This example is representative of experiments done with two groups of laboratory rats to compare the proteins in the blood plasma of genetically bred hypertensive rats with the proteins present in the blood plasma of normotensive control rats. The results of a discontinuous SDS polyacrylamide gel electrophoretic analysis are illustrated in Figures 1 through 3.

The genetically bred hypertensive rats used in the experiments of which this example is representative were spontaneously hypertensive rats (hereinafter "SHR") bred as set forth in Okamoto, K. and Aoki, K., Japanese Circulation Journal 27: 282-293, 1963, and were obtained from the National Institutes of Health, Bethesda, Maryland, U.S.A. The normotensive control rats were the genetic parents of the SHR, namely Wistar Kyoto rats, obtained from the same source as the SHR rats. The Wistar Kyoto normotensive control rats will be referred to hereinafter as "WKYN".

Both groups of rats were maintained on Purina Lab Chow and water ad libitum. Direct arterial pressure was measured through an indwelling Teflon-Tygon catheter placed in the left common carotid artery of the rats. Concerning the two rats whose gels were selected for this Example, the blood pressure of the SHR rat was 212/134 mm Hg systolic/diastolic and the blood pressure of the WKYN rat was 134/100 mm Hg. Based on the great difference between these values, it is readily apparent that the SHR rat was hypertensive. The hypertension was caused by genetic factors. Accordingly, the SHR rat is considered to be a model of human hypertension and, more particularly, a model of human essential hypertension.

Blood was obtained from conscious rats through the carotid artery catheter and placed into cold heparinized tubes. Plasma and cellular components were separated by centrifugation and the plasma was stored at $-80^\circ$C. The

plasma proteins were resolved by discontinuous SDS poly-
acrylamide gel electrophoresis using the apparatus and
compositions described hereinbefore and set forth more
particularly as follows.

The resolving gel comprised acrylamide in an exponential gradient from 8 to 25% prepared by standard techniques using a 10 ml mixing volume. Tris-HCl, pH 8.8, was
present in a concentration of 0.375M. SDS concentration
was 0.1% and glycerol was present in exponential gradient
concentrations from 0 to 10%.

The spacer gel included 6% acrylamide, 0.125M Tris-
HCl at pH 6.8, and 0.1% SDS.

The sample buffer had the following ingredients
whose percentages are expressed as the final concentration
diluted with an equal volume of the sample: 0.025 Tris-
HCl at pH 6.8, 5% glycerol, and 2% SDS.

The electrode buffer contained 3.0 g Tris, 14.4 g
glycine, 0.1% SDS and water to make 1 liter. The electrode buffer had a final pH 8.3.

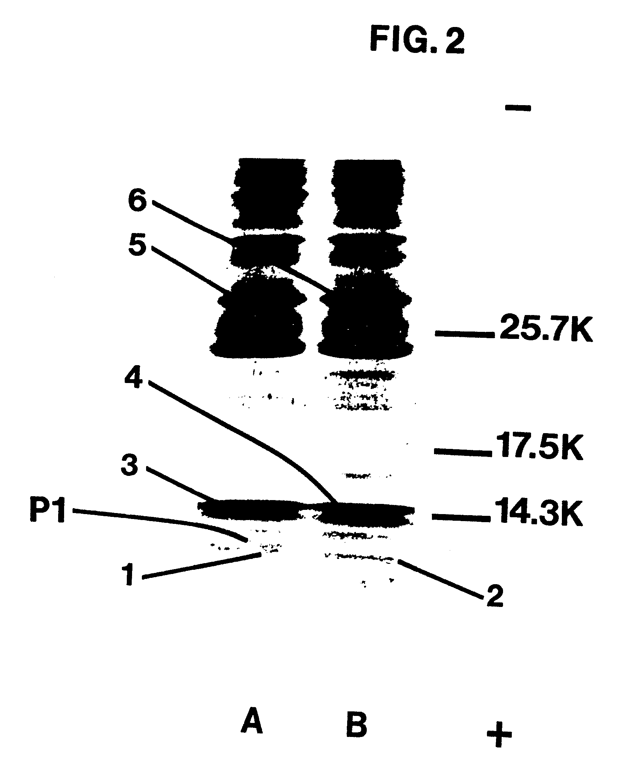

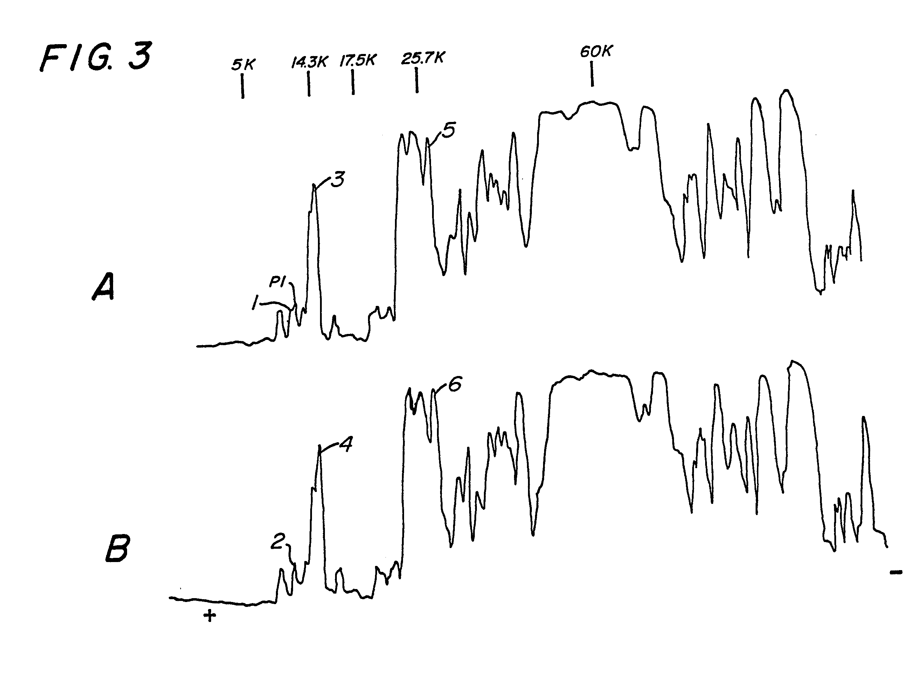

The blood plasma sample solutions were prepared
by diluting the plasma 1:10 with deionized water and then
mixing the diluted samples one to one with the sample
buffer. 2-mercaptoethanol to a final concentration of 5%
was added to the sample solution and this solution was
boiled for 2-5 minutes in a capped tube. An aliquot of
about 40 μl of sample solution from each rat was electrophoresed in a vertical orientation at a constant current
of about 10 mA overnight. The results of the two electrophoresed samples are illustrated in Figure 1, which
is a photograph of the actual gels obtained. Figure 2 is
an enlargement of the left-hand portion of Figure 1 showing the area of primary interest, and Figure 3 is a scan
of a transparency of the photograph of Figure 1 using a
soft laser scanning densitometer (Biomed Instruments,
Inc.) in the high resolution mode.

With reference to Figures 1 and 2 wherein like letters and numerals represent like elements, gel A is the gel of the plasma sample from the SHR rat. Gel B is the gel of the sample from the WKYN rat. Various marker proteins having known molecular weights were electrophoresed simultaneously adjacent to the samples so that relative molecular weight of the proteins in the samples could be determined by interpolation. The marker proteins included insulin, represented by "5K" (5K indicates a molecular weight of about 5,000 daltons); lysozyme at 14.3K; tobacco mosaic virus coat protein at 17.5K; chymotrypsinogen at 27.5K; and catalase at 60K. These marker proteins were used as standards throughout all examples contained herein except for Examples 2 and 3.

In addition to the marker proteins identified by their relative molecular weights on the right-hand side of the photographs, several additional indicator proteins present in the sample solution were used to help determine the relative molecular weight of the protein or proteins associated with hypertension in the samples. The indicator proteins, generally indicated in the Figures by numerals, are generally represented on the gels and the photographs of the gels by relatively dark bands for which relative molecular weights may be easily interpolated.

Reviewing gel A and gel B in Figures 1 and 2, it is apparent that they are substantially identical except for one horizontal band P1 which is present in gel A but not in gel B. Protein band P1 has been found to be representative of the protein associated with hypertension. Thus, the only apparent difference between gel A and gel B is the existence of protein band P1, and the only apparent difference between the two rats is that rat A has genetically derived hypertension while rat B is normotensive. Protein bands 1 and 2 represent the first same indicator protein used to determine the relative molecular weight of P1. Proteins bands 3 and 4 represent the same second indicator protein. Protein bands 5 and 6 represent the same third indicator protein. The indicator protein represented by bands 1 and 2 has a molecular weight of about 12,400 daltons. The protein represented by bands 3 and 4 has a molecular weight of 15,300 daltons. The indicator protein represented by bands 5 and 6 has a molecular weight of about 27,500 daltons. Using the molecular weight of the indicator proteins and the marker proteins, the calculated relative molecular weight (hereinafter "MWr") of Pl is about 12,800.

The word "about" when used herein with respect to molecular weights, means $\pm$ 10%. Thus, when "about" is used, it is used because the analytical technique in combination with the calculation for determining the molecular weights of the proteins gives a result that is precise within a range of approximately 10%.

As used herein, the term "relative molecular weight" or "MWr" means the molecular weight of a protein based upon the relative position of its representative protein band with respect to the protein bands representative of the indicator proteins and the marker proteins. Because the molecular weight is not absolute, the unit "daltons" is not used with respect to "relative molecular weight" or "MWr".

It has been determined that protein band Pl does not represent any of the proteins usually considered to be related to hypertension, namely renin, renin-substrate or angiotensin. This conclusion was reached on the basis of molecular weight determinations. Thus, while the protein associated with hypertension represented by band Pl has a MWr of about 12,700, the molecular weight of renin is about 37,000 to about 43,000 daltons. The molecular weight of renin-substrate is species dependent and ranges from about 56,000 to 110,000 daltons. The molecular weight of angiotensin 1 and 11 are 1457 and 1171 daltons, respectively. Accordingly, Pl appears to be a band representative of a previously unknown protein associated with hypertension.

Figure 3 is a scan of a transparency of the gel of Figure 1 using a soft laser scanning densitometer. With this instrument, the density of the protein bands in the transparency can be measured. The scan is labeled to correspond with the labeling of Figure 1 and Figure 2. Thus, peak 1 and peak 2 of Figure 3 represent the same protein represented by bands 1 and 2 in Figure 1. Likewise with bands 3, 4 and 5, 6. Clearly, there is a difference in scan A and scan B in Figure 3. Peak P1 of scan A is not completely separate from peak 1 of scan A because the protein represented by peak P1 is not well resolved from the protein represented by peak 1. Nevertheless, it is clear that there is a qualitative difference between the respective areas of scan A and scan B in the vicinity of peak P1. The scan of Figure 3 highlights the existence of the protein associated with hypertension as represented by protein band P1 in gel A for the SHR rat in Figures 1 and 2.

## Example 2

This example is representative of a series of experiments comparing the proteins in urine samples from the SHR genetically hypertensive rat and the normotensive WKYN rat. Photographs of electrophoretic gels reveal the existence of a protein associated with hypertension present in the gel of the urine sample of the SHR rat which is not present in the otherwise substantially identical gel of the WKYN rat.

The SHR rats and the genetically parental age-matched WKYN rats were obtained from the National Institutes of Health, and were maintained on Purina Lab Chow and water ad libitum. Urine was collected overnight and stored at -80°C. An indwelling Teflon-Tygon catheter was placed in the left common carotid artery of each rat. Direct arterial blood pressure was measured through the catheter in conscious rats.  The SHR rat had a blood pressure of 210/164 mm Hg and the WKYN rat had a blood pressure of 124/100 mm Hg.  Thus, it is clear that the SHR rat was hypertensive and the WKYN rat was normotensive.

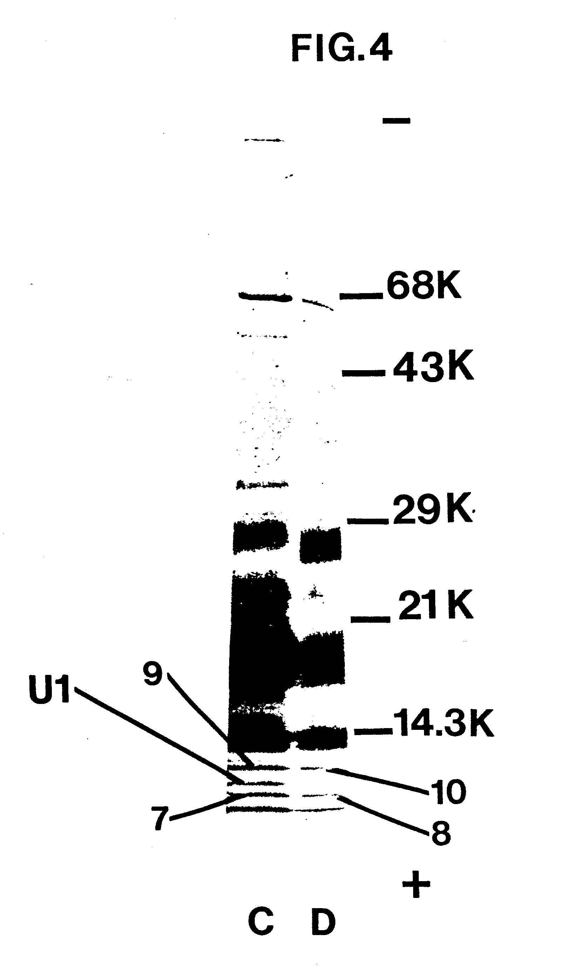

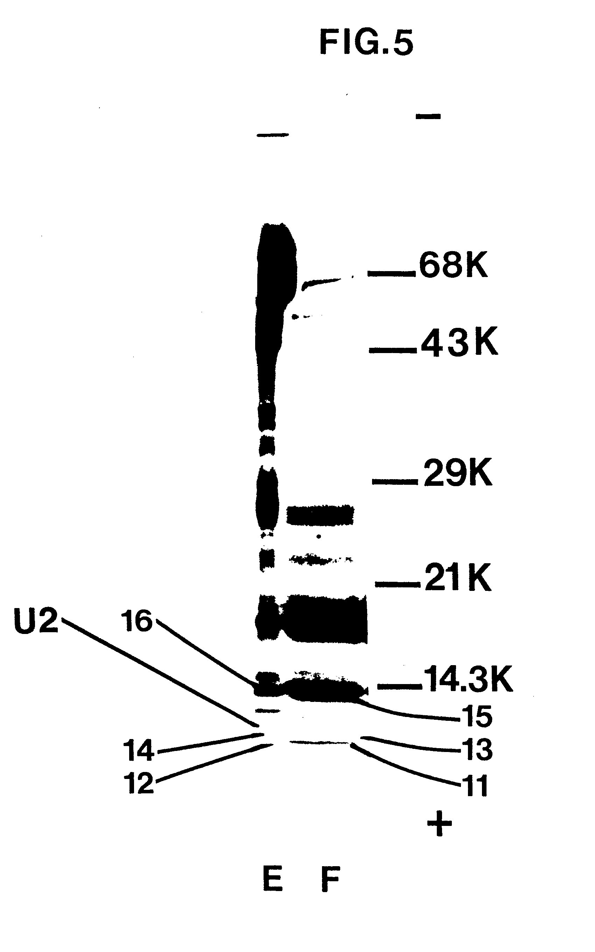

Proteins from unconcentrated urine samples were resolved by discontinuous SDS polyacrylamide gel electrophoresis.  Gels with vertical exponential concentration gradients of acrylamide (from 12 to 25% using a 10 ml mixing volume) were prepared.  The composition of the resolving gel, spacer gel, electrode buffer and sample buffer were the same as those set forth in Example 1 as were the electrophoresis conditions except as described below.  An aliquot of about 40 μg of urine protein from the urine sample solution from each rat was prepared by mixing equal volumes of urine and sample buffer (50 μl of sample solution from the WKYN rat and 75 μl of sample solution from the SHR rat).  The samples were electrophoresed simultaneously adjacent to a solution containing the marker proteins identified on the righthand side of Figures 4 and 5 by their molecular weights: lysozyme (14.3K), soybean trypsin inhibitor (21K), carbonic anhy-drase (29K), ovalbumin (43K), and albumin (68K).  The results of this electrophoresis are illustrated in Figures 4 and 5.

Figure 4 is a photograph of the gels of the urine sample from the SHR rat (gel C) and from the WKYN rat (gel D).  Upon inspecting gels C and D, it is clear that gel C contains an additional protein band, identified as U1 (between indicator protein bands 7 and 9) when compared with gel D.  Otherwise, gels C and D are substantially identical.  Protein bands 7 and 8 in Figure 4 represent the same first indicator protein having a molecular weight of 10,600 daltons.  Protein bands 9 and 10 represent the same second indicator protein having a molecular weight of 12,200 daltons.  Protein band U1 represents the protein associated with hypertension in the SHR rat having a relative molecular weight of about 11,400 interpolated from the molecular weights of the indicator proteins and marker proteins.

Based on the MWr of the protein represented by band Ul, this protein is not renin, renin-substrate or angiotensin. Accordingly, it is believed that protein band Ul is representative of a previously undiscovered protein associated with hypertension.

Since the rats are substantially identical, except that the SHR rat has genetically derived hypertension, and since the proteins in the urine samples are substantially identical, except for the presence of protein band Ul in gel C, it has been determined that band Ul represents a protein associated with hypertension. No laser scanning densitometer scan was made of a transparency of Figure 4 because it is believed that the existence of protein band Ul is sufficiently clear in Figure 4.

### Example 3

This example is representative of experiments to determine what protein distinctions exist in body fluids, particularly urine in this specific example, from laboratory rats which have been.surgically treated to serve as models of renal hypertension, one form of secondary hypertension.

Male Sprague-Dawley rats were used for this experiment. They were maintained on standard Purina Lab Chow and water ad libitum. Arterial pressure was measured through an indwelling Teflon-Tygon catheter inserted in the left common carotid artery. One group of rats was surgically treated by the total ligation of the aorta between the renal arteries and just below the origin of the superior mesenteric artery to produce an experimental model of severe renal hypertension. These rats which were surgically treated to be experimental models of renal hypertension will be referred to hereinafter as "ERH" rats. The surgical method is believed to be known to those of ordinary skill in the art and if further details concerning the procedure are desired, attention is directed
to Fernandes, M., Onesti, G., Weder, A., Dykyj, R., Gould,
A.B., Kim, K.E. & Swartz, C., Journal of Laboratory and
Clinical Medicine, 87: 561-567, 1976; Fernandes, M.,
Fiorentini, R., Onesti, G., Bellini, G., Gould, A.B.,
Hessan, H., Kim, K.E. & Swartz, C., Clinical Science &
Molecular Medicine, 54: 633-637, 1978; and Bellini, G.,
Fiorentini, R., Fernandes, M., Onesti, G., Hessan, H.,
Gould, A.B., Bianchi, M., Kim, K.E. & Swartz, C., Clinical
Science, 57: 25-29, 1979.

A group of age-matched male Sprague-Dawley rats were
used as controls. To have a fair comparison, the control
rats were subjected to a sham operation in which they were
incised, and the aorta beween the renal arteries below the
superior mesenteric artery was manipulated, but not
ligated. After 40 days, aliquots of urine samples were
taken from both groups of rats and subjected to discontinuous SDS polyacrylamide gel electrophoresis using the
same gel and buffer compositions and conditions as in
Example 2. The sizes of the samples were adjusted to
provide easily visible protein bands in the region of
interest, namely about 10,000 daltons to about 17,000
daltons. 20 μl of sample solution from the EHR rat and 50
μl of sample solution from the normotensive rat were
used. The same marker proteins were used as were used in
Example 2.

Two of the gels that were photographed comprise
Figure 5.

Gel E shows the resolution of the urine proteins in
the ERH rat 40 days after the operation to make it a renal
hypertensive model. The ERH rat had a blood pressure of
228/152 mm Hg. Gel E shows the resolution of the urine
proteins in the control rat 40 days after the sham operation. The control rat had a blood pressure of 128/104 mm
Hg.

The urine from the ERH rat (gel E) contained large amounts of protein including high molecular weight species, such as albumin, having a molecular weight of about 68,000 daltons. The urine of the normotensive control rat contained only small amounts of protein species with molecular weights above 25,000 daltons. This may indicate some defect in the filtration process in the ERH rats. However, the protein composition of the urine of the normotensive control and ERH rats is similar for protein species below 25,000 daltons except as indicated below.

The urine from the ERH rat (gel E) contains only one additional protein band labeled U2 when compared with the gel from the normotensive control rat (gel F) in the range below 25,000 daltons.

The calculated MWr of the protein represented by band U2 is 11,400 based on the molecular weights of the marker proteins and indicator proteins. Protein bands 11 and 12 represent the same first indicator protein having a molecular weight of 10,600 daltons. Protein bands 13 and 14 represent the same second indicator protein having a molecular weight of 11,200 daltons. Protein bands 15 and 16 represent the same third indicator protein having a molecular weight of 14,300 daltons.

Because Figure 5 clearly indicates the existence and relative location of protein band U2, there is no corresponding scan of a transparency of Figure 5 using a laser scanning densitometer.

Based on the MWr of U2, this protein associated with hypertension is not renin, renin-substrate or angiotensin. Accordingly, the logical conclusion is that protein band U2 is representative of a protein associated with hypertension.

## Example 4

This example is representative of experiments directed to the detection and identification of proteins associated with hypertension in blood plasma of laboratory rats surgically treated to be models of renal hypertension. The surgical technique was the same as the technique described with respect to Example 3. One additional modification was used in this example as described hereinafter.

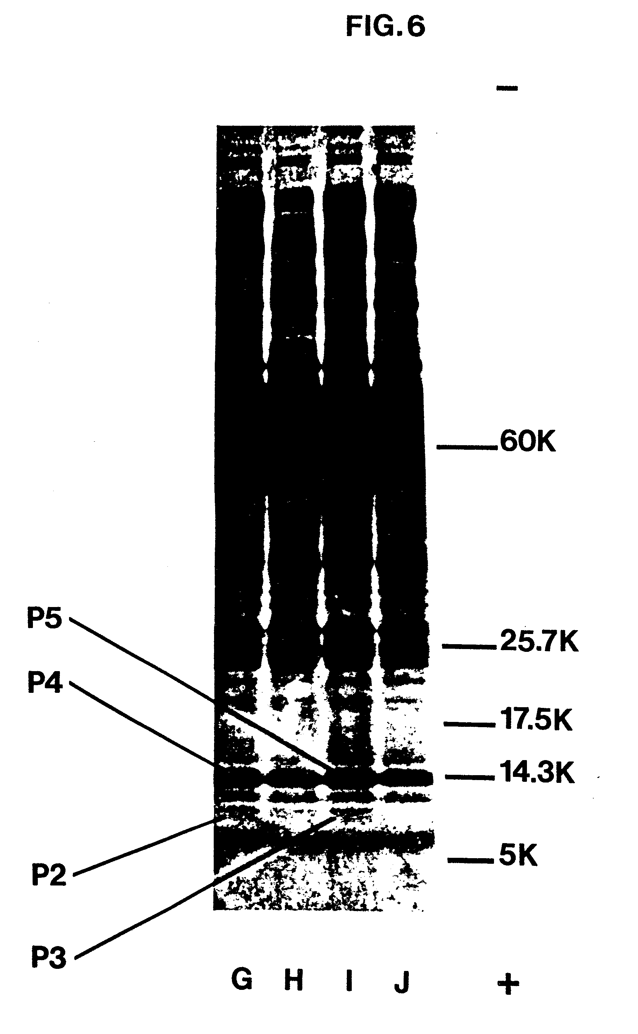

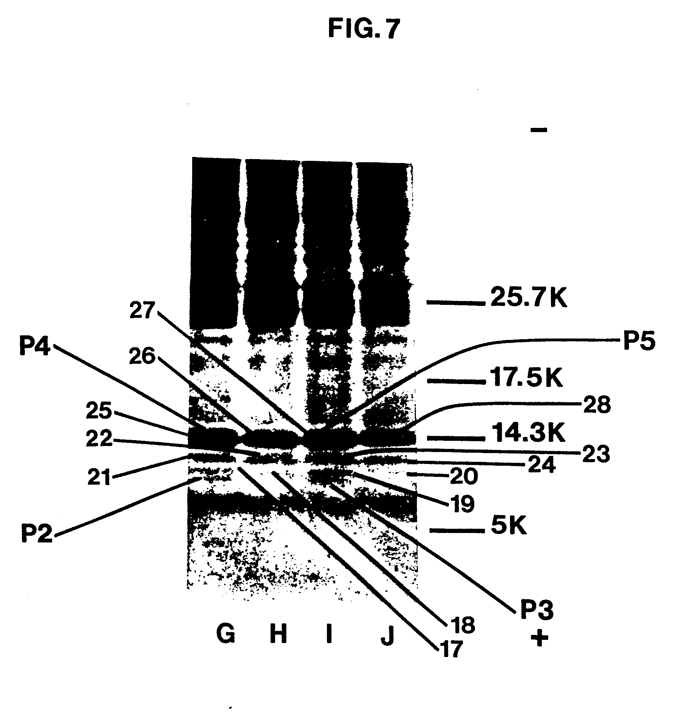

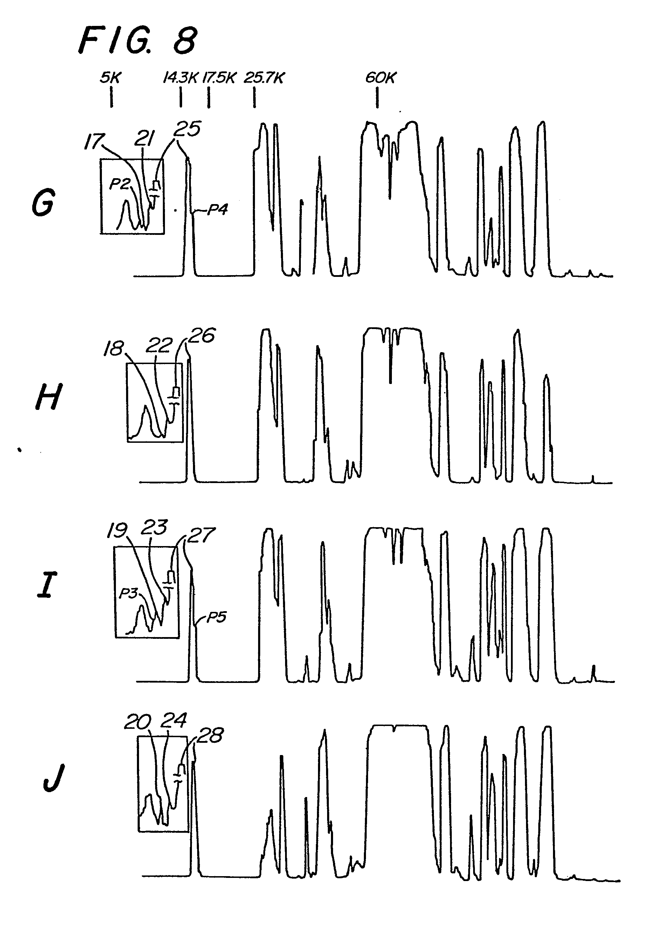

Male Sprague-Dawley rats were used and 40 days after being surgically treated as in Example 3, plasma samples were taken, diluted 1:10 with deionized water and the diluted plasma mixed with an equal volume of sample buffer. The blood plasma samples were prepared as described in Example 1. The electrophoretic conditions and compositions were also the same as those in Example 1, including the use of the same five marker proteins. The proteins in aliquots of about 40 μl of sample solutions of blood plasma of the rats were resolved by discontinuous SDS polyacrylamide gel electrophoresis producing gels which were photographed and are reproduced herein as Figures 6 and 7. A scan of Figure 6 using a soft laser scanning densitometer is reproduced in Figure 8. Like letters and numerals indicate like elements throughout Figures 6, 7 and 8. Figure 7 is an enlargement of the relevant areas of interest of Figure 6 and will be referred to primarily in this description because of space limitations for labeling the elements in Figure 6.

With reference to Figure 7, gel G shows the resolution of proteins in blood plasma of a first ERH rat 40 days after surgical treatment. This rat had a blood pressure of 250/156 mm Hg.

Gel H shows the resolution of proteins in blood plasma of the same rat as in gel G after the surgical removal of the ischemic left kidney resulted in the normalization of blood pressure to a value of 144/100 mm Hg. Thus, gels G and H are of the same rat when it was hypertensive and when it was normotensive, respectively.

Gel I shows the resolution of the proteins in the blood plasma of a second EHR rat 40 days after the surgical treatment. Its blood pressure was 256/172 mm Hg.

Gel J shows the resolution of proteins in the blood plasma of a sham-operated normotensive rat having a blood pressure of 134/96 mm Hg 40 days after the operation. The molecular weights of indicator proteins were also calculated to aid in the determination of the MWr of the proteins associated with hypertension represented by the additional protein bands in the region below 25.7K in gels G and I of the ERH rats.

Comparing gels G, H, I and J as shown in Figure 7, two additional protein bands can be detected and identified in gels G and I. Thus, gel G has an additional protein band labeled P2 which corresponds to a protein band P3 in gel I. Protein band P4 in gel G corresponds to protein band P5 in gel I. Further reference may be had to the scan of Figure 8 (where the peaks are numbered to correspond with the bands of the gels of Figure 6) and particularly to the higher resolution portions in the boxes adjacent to the main scans. The determination of the relative molecular weights of these proteins associated with hypertension (P2, P3, P4 and P5) is more difficult because of the unusual, but characteristic behavior of these proteins associated with hypertension as will be set forth more fully hereinafter with respect to Example 7.

Bands P2 and P3 are believed to represent the same first protein associated with hypertension with a calculated MWr of about 10,900. Bands P4 and P5 are believed to represent the same second protein associated with hypertension with a MWr of about 14,500. Protein bands 17, 18, 19 and 20 represent the same first indicator protein having a molecular weight of 11,500 daltons. Protein bands 21, 22, 23 and 24 represent the same second indicator protein having a molecular weight of 12,600 daltons. Protein bands 25, 26, 27 and 28 represent the same third indicator protein having a molecular weight of 14,200 daltons.

Based upon molecular weight determinations, the protein or proteins represented by bands P2 and P4 in gel G and by bands P3 and P5 in gel I are not renin, renin-substrate or angiotensin.

This Example is important because it demonstrates that there is very little doubt that protein bands P2 and P4 (and the similar if not identical protein bands P3 and P5, respectively) represent proteins associated with hypertension. This is because the same animal was tested when it was hypertensive and when it was normotensive. When it was hypertensive, its blood plasma included the protein or proteins represented by bands P2 and P4 in gel G. When it was normotensive, bands P2 and P4 were absent in gel H. Thus, almost all other causes for the existence of bands P2 and P4 have been eliminated by testing the same animal.

Based on the unusual migration behavior of these proteins associated with hypertension as explained with regard to Example 7, protein bands P2 and P4 on gel G and protein bands P3 and P5 on gel I may represent the same, single protein associated with hypertension. Alternately, bands P2 and P4 may represent related but separate proteins associated with hypertension. Bands P2 and P4 may represent separate, unrelated proteins, both of which are associated with hypertension. The inventors are uncertain which of these theories is or are correct. The fact is, however, that protein bands P2, P3, P4 and P5 exist in hypertensive rats, but not in normotensive rats.

It appears that the protein represented by band U2 in Figure 5 is substantially similar to the protein represented by protein band U1 illustrated in Figure 4. The protein represented by protein bands P3 and P5 of Figures 6 through 8 (the same protein may also be represented by bands P2 and P4 of Figures 6 through 8 as discussed hereinbefore) may be related to the protein represented by protein band P1 of Figures 1 through 3.

Thus, in laboratory rats, it appears that the existence of proteins associated with hypertension in urine and blood plasma indicates a rat is hypertensive, but may not indicate whether the hypertension is genetic hypertension or renal hypertension. This conclusion is not absolute, however. Nevertheless, at least in body fluids of laboratory rats, it is apparent that a protein in a hypertensive rat which is not present in a normotensive rat where the protein has a MWr of about 10,000 daltons to about 17,000 daltons and, more particularly, from about 10,500 daltons to about 16,000 daltons, is a protein which is associated with hypertension.

## Example 5

This Example is representative of experiments conducted using blood plasma from human patients with essential and secondary hypertension compared to blood plasma from normotensive human subjects.

Blood samples were obtained from hypertensive patients attending an out-patient clinic and from normotensive subjects within a similar age range. Two patients, for this Example, had secondary hypertension (one with renal parenchymal disease and one with renovascular disease) but neither had a family history of essential hypertension. The remaining hypertensive patients for this example all had essential hypertension, secondary hypertension being excluded by the history, clinical examination, urinalysis, serum electrolytes, urea nitrogen and serum creatinine. Rapid sequence pyelography and renal arteriography were preformed, when appropriate, to diagnose renovascular disease.

Venous blood was withdrawn into Vacutainer receptacles containing EDTA and chilled in ice immediately. Plasma and cellular components were separated by centrifugation at 4°C. The plasma was stored at -80°C. Samples for electrophoresis were prepared as set forth in Example 1. Aliquots of about 40 $\mu$l of the samples were then subjected to discontinuous SDS polyacrylamide gel electrophoresis under the conditions and using the compositions
set forth in Example 1, including the simultaneous electrophoresis of the same marker proteins.  The gels were
stained with 0.1% Coomassie Brilliant Blue R 250, and 50%
methanol and 10% acetic acid, and destained using 10%
methanol and 10% acetic acid.

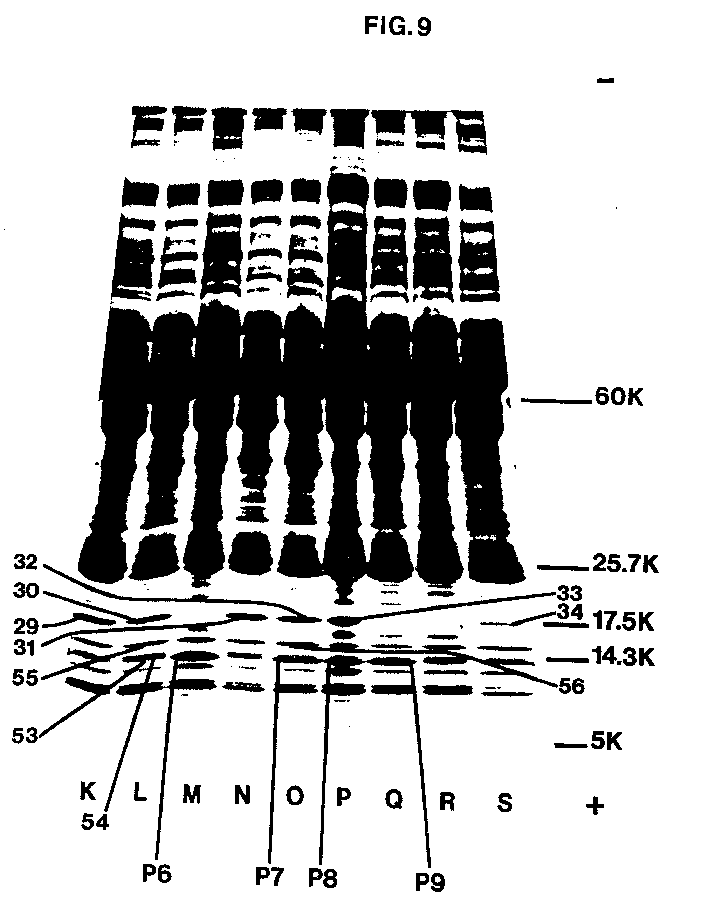

The results of the electrophoretic resolution of the
proteins in the blood plasma samples of nine subjects are
illustrated in Figure 9.  For ease of understanding, the
relevant information is contained in the following table:

### Table 1

| Subject | Condition | Protein Band Assoc. With Hypertension |
|---|---|---|
| K | RPD(1) | No |
| L | NT(2) | No |
| M | EH(3) | P6 |
| N | NT | No |
| O | EH | P7 |
| P | EH | P8 |
| Q | EH | P9 |
| R | NT | No |
| S | RVD(4) | No |

(1)  Renal parenchymal disease and secondary hypertension
     with no family history of essential hypertension
(2)  Normotensive
(3)  Essential Hypertension
(4)  Renovascular disease and secondary hypertension with
     no family history of essential hypertension

It can be seen by examining the gels of Figure 9 and
the data summarized in Table 1 that the blood plasma of
persons having essential hypertension contains an additional protein band representative of a protein having a relative molecular weight of about 14,000. Thus, the gels of subjects M, O, P and Q, all with essential hypertension, have protein bands identified as P6, P7, P8 and P9, respectively. These protein bands are representative of the same protein associated with essential hypertension. Thus, by detecting and identifying the presence of these bands, it is possible to differentiate persons having essential hypertension (M, O, P and Q) from normotensive subjects (L, N and R) and also from persons having secondary hypertension, such as hypertension associated.with renal parenchymal disease or renovascular disease (K and S, respectively).

The protein represented by protein bands P6 through P9 has been concluded to be a protein associated with human essential hypertension because it is absent in subjects who do not have a family history of essential hypertension. The presence of protein bands P6 through P9 does not appear to be correlated with age, sex or race. In hypertensive patients, such as subjects K and S whose blood plasma does not contain this protein, the cause of hypertension is non-genetic or non-familial (hence, non-essential), and is due to a variety of secondary causes involving the kidney, kidney artery, adrenal gland or the like.

Protein bands P6 through P9 appear as widened dark areas adjacent to two very closely spaced protein bands, both of which are present in all of the subjects tested. This is because the lower band is representative of two proteins, one of which is associated with essential hypertension. Thus, in gels M, O, P and Q there are actually three proteins represented by the bands in the region of about 14.3K. The protein associated with hypertension which is represented by each of bands P6 through P9 is not resolved because of a unique but characteristic migration behavior which is displayed during electrophoresis on gels containing horizontal gradients of concentration of polyacrylamide as will be pointed out hereinafter with respect to Example 7. Nevertheless, it is believed that one of ordinary skill in the art would have no trouble discerning the existence of proteins represented by protein bands P6 through P9 once alerted to look for these.

The protein represented by protein bands P6 through P9 is not renin, renin-substrate or angiotensin based upon its relative molecular weight. While its pathophysiologic function is unknown at this time, the protein represented by bands P6 through P9 is important as a marker protein for differentiating essential hypertension from normotension generally, and essential hypertension from secondary hypertension more specifically.

There are some additional differences between the gels of Figure 9. The differences include the presence of protein bands representative of a protein having a MWr of about 17,500. Band 29 is present in gel K, band 30 is present in gel L, band 31 is present in gel N, band 32 is present in gel O, band 33 is present in gel P and band 34 is present in gel S. The protein represented by bands 29 through 34 does not correlate with sex, race, age or blood pressure. Accordingly, it is merely an inconsistent variation and should not be mistaken for bands P6 through P9 representative of the protein associated with hypertension.

Although the gels of two other subjects tested do not comprise a part of Figure 9, they are worthy of discussion. Two patients with atherosclerotic renal artery stenosis (resulting in secondary hypertension) superimposed on long-standing essential hypertension were tested. Blood plasma samples from these two patients were subjected to discontinuous SDS polyacrylamide gel electrophoresis as set forth in this Example. The gels of these two patients contained protein bands corresponding to bands P6 through P9. Thus, even when there is evidence of secondary hypertension, so long as a portion of the hypertension appears to be from unknown or genetic factors (that is, essential hypertension), a protein band representative of a protein associated with essential hypertension will exist.

### Example 6

This Example compares the protein content of blood plasma from human patients with essential and secondary hypertension and from normotensive human subjects using a uniform concentration of resolving gel in a discontinuous SDS polyacrylamide gel electrophoretic analysis.

As in Example 5, blood plasma samples were obtained from patients with essential hypertension and secondary hypertension, as well as from normotensive volunteers within the same age range. One normotensive subject (T) was a 30 year old woman with a strong family history of essential hypertension.

Aliquots of about 40 $\mu$l of the samples were subjected to discontinuous SDS polyacrylamide gel electrophoresis in a vertical orientation in which the resolving gel had a uniform concentration of 13% acrylamide. Other than this difference, the remaining electrophoresis compositions and conditions were the same as those in Example 5.

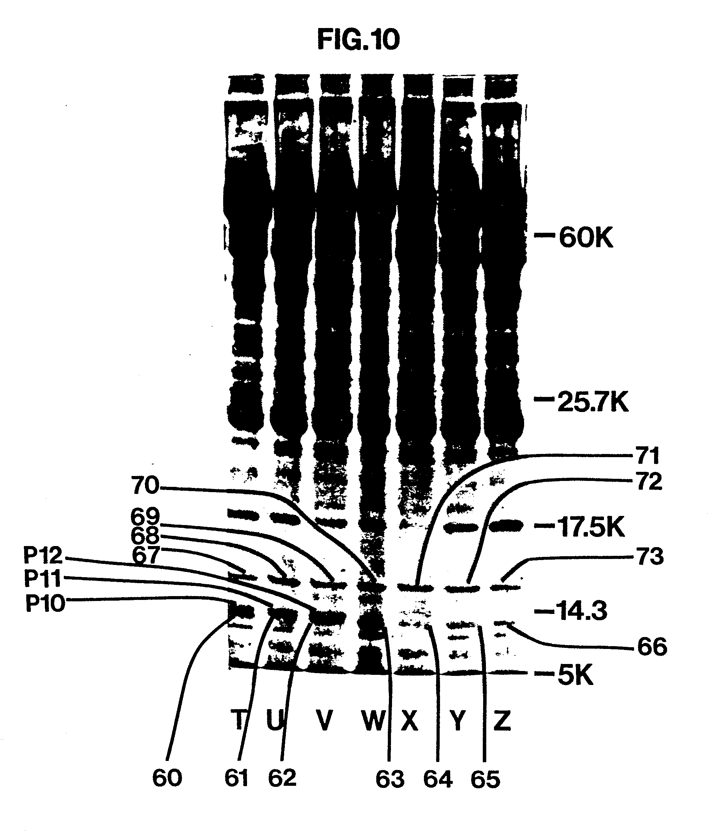

Further data concerning the subjects and the results of the electrophoretic analysis are summarized in Table 2 and illustrated in Figure 10.

<div align="center">Table 2*</div>

| Subject | Condition | Protein Band Assoc. With Hypertension |
|---------|-----------|---------------------------------------|
| T | NT** | P10 |
| U | EH | P11 |
| V | EH | P12 |
| W | NT | No |
| X | RPD | No |
| Y | RVD | No |
| Z | NT | No |

---

\* The abbreviations in this Table are the same as those in Table 1.

\*\* Family history of essential hypertension.

The results of this Example correlate with the results of Example 5. Thus, subjects U and V, both with essential hypertension, have a protein band representative of a protein associated with hypertension. Band P11 is found in the gel of patient U and band P12 is found in the gel of patient V. Corresponding bands are not found in the gels of any other subjects (except in the gel of subject T to be discussed hereinafter), whether they have secondary hypertension (X and Y) or are normotensive (W and Z). Accordingly, it is believed that the protein represented by bands P11 and P12 is a protein associated with essential hypertension.

An inspection of Figure 10 reveals the presence of an additional protein band P10 in the gel of subject T, the normotensive young woman with a family history of essential hypertension. Band P10 is in the same region of relative molecular weight as protein bands P11 and P12 which represent a protein associated with the hypertension. The presence of band P10 in the gel of subject T is the only discernible difference in the gel of subject T compared to the gels of the subjects with secondary hypertension (X and Y) and compared to the normotensive subjects without a family history of essential hypertension (W and Z). Accordingly, because of the presence of band P10 in the gel of subject T, it is believed that band P10 is representative of a protein associated with essential hypertension, and may be a link to the genetic or familial cause of essential hypertension. Further, based on the existence and location of band P10 in the gel of subject T, it is believed that band P10 represents the same protein associated with essential hypertension as represented by bands P11 and P12 in the gels of subjects U and V, respectively.

Thus, it is believed that the data supports the conclusion that the detection and identification of this protein may be used as a marker protein for determining the predisposition of a person to essential hypertension. With this information, a normotensive person having this protein can be carefully monitored and/or take preventive actions with respect to the development of essential hypertension.

Protein bands 60 through 66 represent the same first indicator protein having a molecular weight of 14,200 daltons. Protein bands 67 through 73 represent the same second indicator protein having a molecular weight of 15,900 daltons. Based on the molecular weight of the indicator proteins and the marker proteins, the protein represented by bands P10, P11 and P12 has a calculated MWr of 14,700. The protein represented by bands P10 through P12 displays the same relationship to essential hypertension as the protein represented by protein bands P6 through P9. In light of the unusual migratory behavior of the protein associated with hypertension (see Example 7), it is believed that these two proteins are identical. Therefore, the conclusions reached with respect to the protein represented by bands P6 through P9 and bands P10 through P12 apply interchangeably.

Another preferred method of detecting protein associated with hypertension is to use a horizontal polyacrylamide gel concentration gradient technique wherein there
is in the horizontal graident gel a protein band representative of the protein associated with hypertension which
displays migration of the protein associted with hypertension from a first position corresopnding to a first relative molecular weight to a second position corresponding
to a second relative molecular weight greater than the
first relative molecular weight.  This may be displayed
on the horizontal gradient gel by a protein band which
crosses over or approaches an adjacent protein band.  This
is a very unusual occurrence and is characteristic of the
protein associated with hypertension having a relative
molecular wight range of about 10,000 to about 17,000.

### Example 7

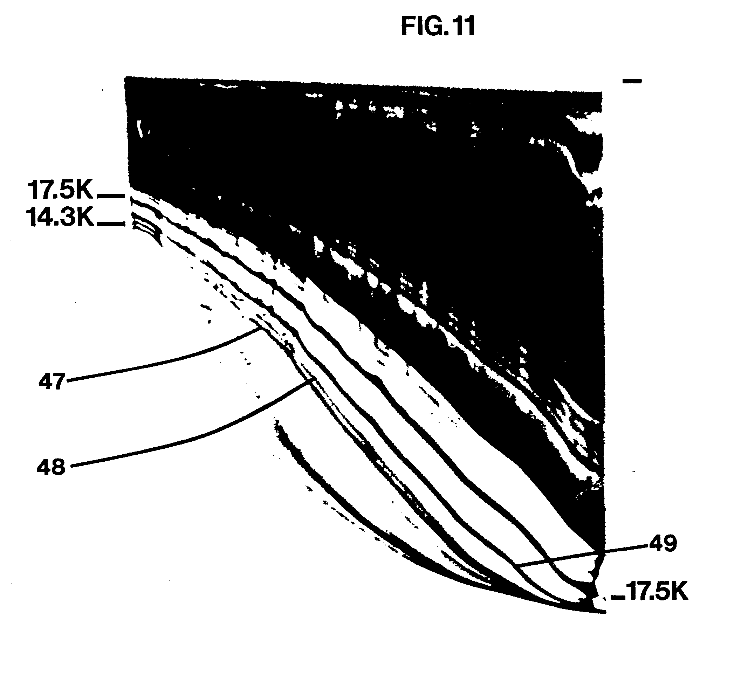

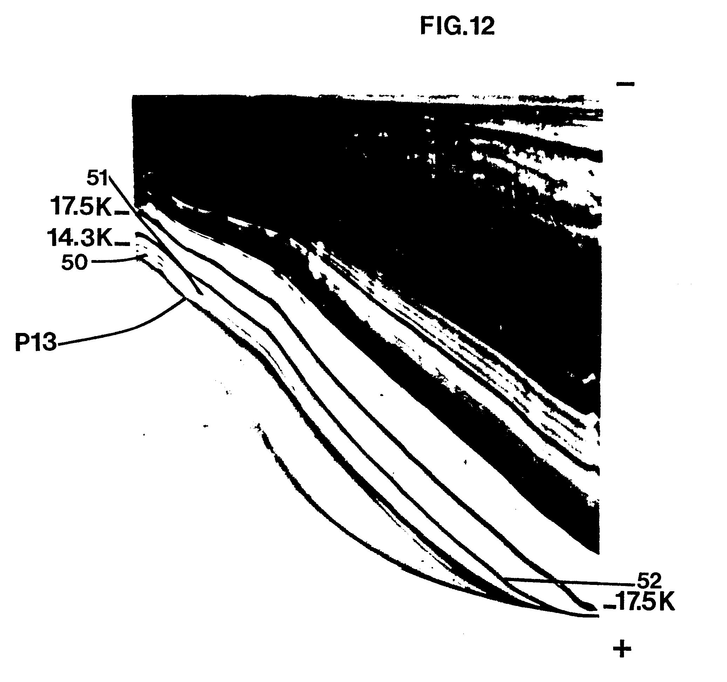

This Example is representative of experiments which
demonstrate the unusual migrating characteristics of the
proteins associated  with hypertension in the urine and
blood plasma of rats and humans as referred to hereinbefore with respect to SDS polyacrylamide gel electrophoresis on gels containing horizontal gradients of concentration of acrylamide.  Figures 11 and 12 relate to this
Example.

The equipment, resolving gel composition, spacer
gel composition and buffer electrode composition are the
same as in Example 5.  Horizontal gradient gels were prepared.  A horizontal gradient gel is a gel in which the
acrylamide concentration gradient varies from side to
side, rather from top to bottom.  In a vertical gradient
gel, the high concentration of acrylamide is on the bottom
and the low concentration of acrylamide is on the top.
In the horizontal gradient gels illustrated in Figures 11
and 12, the high concentration of acrylamide is on the
left and the low concentration of acrylamide is on the
right.  Thus, the gel plates used in producing the gels of Figures 11 and 12 were rotated 90° clockwise before being subjected to electrophoresis when compared to Figures 1 and 2, for example. It is only necessary to make sure that the spacer strip is transferred to the open vertical side of the gel plate assembly and sealed before the gel plates are rotated.

The sample solution was prepared as follows. Blood plasma was obtained and treated as in Example 5. 100 μl of the plasma was diluted with 900 μl of deionized water. The diluted plasma was mixed with an equal volume of sample buffer prepared as in Example 1 to form a sample solution. The sample solution (about 2 ml) was electrophoresed as described above in this Example.

Figure 11 is a photograph of a horizontal gradient gel showing the resolution of proteins in the blood plasma of subject L whose vertical concentration gradient gel is reproduced in Figure 9. Subject L was a normotensive subject and the gel of Figure 11 illustrates the usual migration behavior. Band 47 of Figure 11 corresponds to lower band 53 of the doublet band around the 14.3K marker of Figure 9. Band 48 of Figure 11 corresponds to the upper band 54 of the doublet band in Figure 9 around the 14.3K marker. Band 49 of Figure 11 corresponds to the next higher band 55 on Figure 9 between the marker proteins at 14.3K and 17.5K. On SDS polyacrylamide gels, the $R_f$ (the distance of migration of a protein relative to the ion front) is inversely related to the polyacrylamide concentration at all concentrations of the polyacrylamide in the gel. The $R_f$ is inversely related to the log MW (logarithm of the molecular weight) of the protein. For any given concentration of polyacrylamide, there is a range of molecular weights which displays a linear inverse relationship between $R_f$ and log MW so that the molecular weight of the protein can be determined based upon the $R_f$. Proteins with molecular weights above and below that range deviate from the linear relationship so that the accurate molecular weight of these proteins cannot be determined based upon the particular polyacrylamide gel concentrations involved, and other polyacrylamide gel concentrations must be used for which a linear relationship exists. Usually, as illustrated in Figure 11, where proteins display a linear relationship between $R_f$ and log MW, the ratio of the $R_f$'s of any two proteins will remain constant (subject to experimental variability) at all concentrations of polyacrylamide.

Figure 11 illustrates the usual, expected migratory behavior pattern of proteins throughout the gel. It can be seen that each of the bands 47, 48 and 49 is separated from the other by a space. The slopes of bands 47, 48 and 49 are such that the bands do not approach or cross over an adjacent band or adjacent bands representative of a protein or proteins of higher relative molecular weight.

The migratory behavior of proteins associated with hypertension in a horizontal concentration gradient gel is different from the behavior of other proteins as just described. In the case of proteins associated with hypertension comprising the subject of this invention, the ratio of the $R_f$ of the protein associated with hypertension and the $R_f$ of another protein, such as a marker protein, does not remain constant at all concentrations of polyacrylamide within the range where there should be a linear relationship between $R_f$ and log MW of the protein associated with hypertension. Rather, this ratio varies as the concentration of the polyacrylamide varies. Therefore, the log MW of the protein associated with hypertension is not a linear function of its $R_f$. This is an unusual characteristic specific to proteins associated with hypertension within the range of about 10,000 daltons to about 17,000 daltons. The detection of this previously undetected and unidentified specific characteristic has been made possible by the present invention.

This unusual characteristic is illustrated in Figure 12 which is a photograph of a horizontal gradient gel showing the resolution of proteins in blood plasma of subject 0 in Example 5. Thus, Figure 12 is a horizontal gradient gel of the vertical gradient gel for subject 0 in Figure 9.

Protein band 50 in Figure 12 corresponds to the lower band around 14.3K of gel 0 in Figure 9 (which corresponds in turn to band 53 in gel L in Figure 9). Band 51 in Figure 12 corresponds to the upper band of the doublet in gel 0 of Figure 9 around 14.3K (corresponding, in turn, to upper doublet band 54 of gel L in Figure 9). Band 52 in Figure 12 corresponds to protein band 55 in gel 0 in Figure 9 between the 14.3K and 17.5K marker proteins. As was the case with Figure 11, bands 50, 51 and 52 (which correspond with bands 47, 48 and 49, respectively, in Figure 11) show the same general spacing and slope as bands 47, 48 and 49 of Figure 11. This is as it should be, since the ratio of the $R_f$'s of these proteins remain constant.

The major difference between Figures 11 and 12 is the existence of an additional protein band P13 in Figure 12 and the different mobility characteristics exhibited by protein band P13. Protein band P13 in Figure 12 represents the same protein associated with hypertension that was represented by band P7 in gel 0 of Figure 9.

In addition to the existence of band P13 in Figure 12 as a distinguishing characteristic between Figures 11 and 12, the migration behavior of band P13 is unusual. Thus, at the top of Figure 12, band P13 migrates at a position corresponding to a MWr less than 14.3K and less than the MWr of bands 50 and 51. The protein represented by band P13 has a calculated MWr at the top of the gel of about 13,000. However, in the middle of the gel, band P13 migrated as if it has a higher molecular weight, identical to bands 50 to 51. Band P13 appears to undergo an "inversion" or a "cross-over" with respect to adjacent bands 50
and 51 and approaches band 52.  Toward the bottom of Figure
12, the protein represented by band P13 migrates as if it
has a MWr greater than the MWr of bands 50 and 51.  The
protein associated with hypertension and represented by
band P13 has a calculated MWr of about 15,000 at the bottom
of the gel.  Thus, unlike the other proteins in the gel
which have a constant MWr, the MWr of the protein associated with hypertension appears to change.  The reason why
this occurs is presently unknown.  Nevertheless, the fact
that this change in slope does occur is a further identification factor for the protein or proteins associated
with hypertension.

It must be emphasized that Figure 12 only illustrates
one example of the unusual migratory behavior of a protein
associated with hypertension.  Thus, while protein band
P13 has a slope which approaches or crosses over an adjacent band or bands representative of a protein of higher
relative molecular weight, this is so because of the amount
of the sample electrophoresed in Figure 12.  Thus, if, for
example, 10 μl of blood plasma were electrophoresed rather
than 100 μl, the amount actually electrophoresed in Figure
12, perhaps protein bands 50 and 51 (and maybe even band
52) would not be visible on the gel because the proteins
they represent are present in only very minute quantities.
In that instance, band P13 would not exhibit a "cross-over"
behavior because the reference bands would not be visible.
Nevertheless, the existence of band P13 and its migration
with respect to the other protein bands which are visible
would be an indication of the existence of a protein associated with essential hypertension.

By using the techniques described herein and by
following the preceding examples, as well as the equivalent
techniques mentioned hereinbefore, it is possible to detect
and identify in a mammal's body fluid a protein associated
with hypertension having a relative molecular weight of about 10,000 daltons to about 17,000 daltons; and more specifically, about 10,500 daltons to about 16,000 daltons; and most specifically, about 13,000 daltons to about 15,000 daltons. The protein associated with hypertension may also be characterized by the unusual mobility characteristics described in Example 7.

By detecting and identifying the protein associated with hypertension, it is possible to determine whether a mammal has hypertension, which is a determination that is frequently not possible to make based on blood pressure measurements alone. Further, at least where humans are concerned, the detection of at least one protein associated with hypertension is an indication that the person has or at least is predisposed to essential hypertension. If a person has high blood pressure and the protein associated with hypertension as described herein is absent, it indicates the increase in blood pressure is due to an identifiable cause (secondary hypertension). If a person has high blood pressure and the protein associated with hypertension is present, it indicates that there is at least a genetic predisposition to essential hypertension.

Isolation Of Protein Associated With Hypertension

Now that a protein associated with hypertension has been detected, a program of hypertension treatment and maintenance based on this discovery is possible. Although the structure and specific identification of the protein associated with hypertension has not been determined, the protein has been sufficiently isolated to prepare an immune serum containing antibody to the protein associated with hypertension.

While the specific mechanism relating to the pathophysiologic function of the protein associated with hypertension is unknown at this time, the inventors have demonstrated that the protein associated with hypertension is in some way at least partly responsible for causing hypertension in mammals generally, and essential hypertension in humans. This general function will be referred to throughout this application as the "hypertensive effect" of the protein associated with hypertension.

More specifically, the inventors have demonstrated that the administration to a mammal of immune serum containing antibody to the protein associated with hypertension blocks the hypertensive effect of the protein associated with hypertension. This indicates that the protein associated with hypertension has a function in elevating blood pressure. Thus, the antibody to the protein associated with hypertension is an agent which blocks the interactions and functions of the protein associated with hypertension. Accordingly, any other agent which blocks the interactions and functions of the protein associated with hypertension will lower blood pressure.

The inventors have also conducted experiments relating to the active immunization with respect to the protein associated with hypertension. These experiments have indicated that a protein associated with hypertension, at least from a species of mammal different from the mammal being treated, comprises an antigen that induces the formation, in the mammal being treated, of antibody to the immunizing protein associated with hypertension.

The first step in preparing antibody to the protein associated with hypertension is to sufficiently and substantially isolate the protein associated with hypertension. Although this may be accomplished by many different techniques, depending upon the relative knowledge of the protein associated with hypertension, its structure, its polypeptide or amino acid map, its mechanism of function, etc., with the equipment available to the inventors at the present time, an electrophoretic isolation of the protein associated with hypertension is most convenient. Accordingly, the following specific, non-limiting example illustrates a method of isolating the protein associated with hypertension based on preparative electrophoresis, as opposed to the analytical electrophoretic methods set forth in the previous examples.

### Example 8

In this example, a protein associated with hypertension was isolated from the other proteins contained in a sample of blood plasma from a SHR rat using discontinuous SDS polyacrylamide gel electrophoresis.

The composition of the resolving gel, spacer gel, electrode buffer and sample buffer were the same as those set forth in Example 1, except that an inert spacer in the shape of a comb did not form a number of sample wells to receive different samples in the same gel.

A sample of blood plasma which contained a protein associated with hypertension was obtained from a SHR rat. A sample solution was prepared by diluting the plasma 1:10 with deionized water (by adding 0.1 ml of plasma to 0.9 ml of deionized water). The diluted plasma sample was mixed 1:1 with the sample buffer. 2-mercaptoethanol to a final solution concentration of 5% was added to the sample solution and the solution was boiled for 2-5 minutes in a capped tube.

An aliquot of about 1.6 ml of the sample solution was electrophoresed in a vertical orientation at a current of about 20 to about 25 milliamps overnight. Although about 1.6 ml of the sample solution was used for the present example, from about 1.0 ml to about 3.0 ml of a plasma sample solution can be used conveniently.

After the electrophoresis was complete, a gel was obtained having a pattern of protein bands similar to the pattern of protein bands in gel A in Figure 1. Since the protein bands extended substantially across the entire width of the gel, a vertical strip from the center of the gel was cut out and physically separated from the rest of the gel. The vertical strip was then stained and destained using standard techniques with bromophenol blue and Coomassie Brilliant Blue R 250. The protein bands containing the protein associated with hypertension were then identified by comparing the protein band pattern of the vertical strip to the protein band pattern of similar analytical gels, such as gels A and B as illustrated in Figure 1.

The stained vertical strip was then compared to the remaining unstained portions of the gel from which the vertical strip was removed. Using the stained vertical strip as a guide, horizontal strips were cut from the unstained portions of the gel in the regions adjacent to the location of the protein associated with hypertension. The horizontal strips were cut out to isolate and separate the protein band containing the protein associated with hypertension from the rest of the gel. The horizontal strips containing the bands having the protein associated with hypertension were then transferred to a test tube. After the horizontal gel strips were transferred to the test tube, the remaining portions of the gel (not containing the protein associated with hypertension) were stained to determine that they did not contain the protein associated with hypertension, and thereby to verify that the appropriate protein bands were isolated.

The horizontal gel strips containing the protein associated with hypertension were then homogenized and soaked in 10 mM phosphate buffer pH 7 overnight. There are several standard techniques for homogenizing the gel strips. One technique that was used was to add enough phosphate buffer to cover the gel pieces and then sonicate the mixture with ultrasound until the gel pieces are suspended throughout the buffer. Another exemplary technique is to homogenize the gel strips by passing them through a series of syringe needles (from 18 gauge to 25 gauge, for example). The purpose of the homogenization is to diffuse the protein throughout the suspension.

The suspension was then centrifuged to separate large pieces of acrylamide from the suspension. The supernatant fraction contained the protein associated with hypertension.

The protein associated with hypertension is now sufficiently isolated for use in the preparation of an immune serum containing antibody to the protein associated with hypertension.

Preparation Of Immune Serum

·The preparation of a suitable immune serum will now be described with reference to the following specific, non-limiting example.

Example 9

Equal aliquots of the supernatant fraction containing the protein associated with hypertension obtained in Example 8 and of Freunds adjuvant were mixed together. Aliquots of about 0.5 ml to about 1.0 ml of the freshly prepared supernatant-adjuvant solution were injected subcutaneously into a New Zealand white rabbit. Booster injections of about 0.5 ml to about 1.0 ml were given to the rabbit two weeks after the initial injection.

Blood samples from the rabbit were obtained by cardiac puncture prior to initial immunization and two and six weeks after immunization. Serum was prepared from the blood samples. The serum that was obtained from the blood samples prior to immunization is called preimmune serum. The serum that was obtained subsequent to the immunization is called immune serum because it contains antibody to the protein associated with hypertension.

The existence of antibody to the protein associated with hypertension was determined by observing the formation of precipitates comprising antigen-antibody complexes after capillary diffusion against whole plasma. One-half of a capillary tube contained whole plasma diluted 1:1 with phosphate buffered physiological saline solution in which the other half of the tube contained immune serum diluted 1:1 with phosphate buffered physiological saline solution. Other experiments were done using barbital buffer and 0.1% Triton X-100 as the diluent with the same results. The tube was left undisturbed for a couple of days in a refrigerator.  An immune precipitate was found in the bottom of
the tube.  The immune precipitate was the protein associated with hypertension, since the only fraction of the
blood plasma from the SHR rat used to induce the formation
of antibody was the fraction which contained the protein
associated with hypertension.

### Administration Of Therapeutic Agents

Immune serum obtained as set forth in Example 9 after
the booster injections was injected into several laboratory
rats to determine whether the immune serum would be effective in treating hypertension.  In each case, the blood
pressure of the rats tested was lowered by passive immunity
transfer following the injection of the immune serum.  The
procedures and results are set forth in the following specific, non-limiting example.

### Example 10

Three types of rats serving as models of various
types of hypertension, as well as control rats, were used
in the passive immunity transfer experiments.  The three
types of rats were the SHR, WKYN (see Example 1) and ERH
rats (see Example 3).  All rats were maintained on standard Purina Lab Chow and water ad libitum.  Arterial pressure was measured through an indwelling Teflon-Tygon
catheter inserted in the left common carotid artery.  The
rats were conscious and unrestrained during the continuous
recordation of blood pressure.

The SHR and WKYN rats were age-matched litter mates
having an age of about 30 weeks.  The rats weight about
300 grams to about 400 grams.

The rats were administered bolus injections of three
types of solutions directly into the arterial cannula:
immune rabbit serum containing antibody to the protein
associated with hypertension taken from the SHR rat; pre-
immune rabbit serum, that is, serum taken from the same
rabbit used to make the immune serum, but prior to immunizing the rabbit with the protein associated with hypertension taken from the SHR rat; and physiological saline solution.   Blood pressures were recorded before and after each injection.

Based upon observation of the blood pressures, some of which were not recorded, it became clear that there was no meaningful difference between the pre-injection blood pressure and the post-injection blood pressure for rats injected with the saline solution or the pre-immune serum. Thus, it was determined that injections of the saline solution and the pre-immune serum did not significantly lower or otherwise effect blood pressure in the rats.   The recorded data also support these conclusions.

The other parameters of the experiments and the results obtained are set forth in the following table in which the injections given to a rat were made in the order as indicated in the table:

# TABLE 3

| Animal | Type of Injection | Amount Injected (ml) | Pre-Injection Blood Pressure (mm Hg) | Post-Injection Blood Pressure (mm Hg) | Approx. Duration BP Change (minutes) |
|---|---|---|---|---|---|
| WKYN #1 | Saline | 0.5 | 120/92 | 110/80 | <0.5 |
|  | Preimmune serum | 0.5 | 150/112 | 140/106 | <0.5 |
|  | Immune serum | 0.5 | 132/88 | 116/76 | <0.5 |
| SHR #1 | Preimmune serum | 0.5 | 220/148 | 220/140 | <0.5 |
|  | Immune serum | 0.5 | 212/156 | 200/132 | 0.5 |
|  | Immune serum | 0.5 | 208/172 | 198/160 | 0.5 |
| SHR #2 | Saline | 0.2 | 220/168 | 220/164 | <0.5 |
|  | Immune serum | 0.2 | 216/156 | 192/142 | 1 |
| SHR #3 | Saline | 0.5 | 192/140 | 186/134 | Stable |
|  | Immune serum | 0.2 | 188/144 | 168/112 | 1 |
|  | Immune serum | 0.5 | 182/148 | 160/102 | 2-3 |
| SHR #4 | Immune serum | 0.5 | 216/170 | 200/140 | 2-3 |
| SHR #5 | Saline | 0.5 | 198/144 | 204/144 | <0.5 |
|  | Immune serum | 1.0 | 200/148 | Pressor 216/156 | 0.5 |
|  |  |  |  | Depressor 162/122 | From 1 to 10* |
|  | Immune serum | 0.5 | 208/152 | Pressor 218/160 | 0.5 |
|  |  |  |  | Depressor 180/144 | From 2 to 7 |
|  | Immune serum | 0.8 | 210/150 | Pressor 220/166 | 2 |
|  |  |  |  | Depressor 184/130 | From 2 to 10 |
| ERH | Immune serum | 0.2 | 220/156 | 200/140 | 1-2 |
|  | Immune serum | 0.5 | 216/152 | 194/132 | 1-3 |

*The notation for example "From 1 to 10" means that the blood pressure indicated was noted from the first to the tenth minute after injection.

In normotensive WKYN rats, injections of saline solution and preimmune serum had no significant effect on the blood pressure of the rats. The blood pressure was lower by 10/8 mm Hg and 10/6 mm Hg, respectively. Injection of the WKYN rats with immune serum produced a blood pressure reduction of 16/8 mm Hg. While this is slightly greater than the blood pressure reduction noted after the injections of the saline solution and preimmune serum, it is still not considered a significant hypotensive response. This is particularly true when compared to the marked hypotensive responses noted after injecting the hypertensive SHR and ERH rats with the immune serum.

As indicated above, the injections of saline solution and preimmune serum in the hypertensive SHR rats produced either no change or slight, transient blood pressure reductions up to 6/8 mm Hg. In contrast, after being injected with the immune serum, a clear trend toward marked reductions in blood pressure was noted. The blood pressure reductions ranged from about 10 mm Hg to about 50 mm Hg for both the systolic and diastolic pressures.

In SHR rat #5, the injection of the immune serum caused a short, transient pressor effect, during which the blood pressure was slightly elevated. This effect did not occur in any of the other rats. The cause of this pressor effect is unknown. However, the inventors believe, without wishing to be bound to any theory, that the pressor effect may have been due to the relatively large dose of immune serum injected (initially, 1.0 ml, followed by injections of 0.5 ml and 0.8 ml). Despite the slight pressor effect, it is clear from Table 3 that the injection of the immune serum produced a marked depressor effect a short time after the pressor effect had subsided, which depressor effect extended for a significant duration compared to other dosages.

The injections of the immune serum into the ERH rat, the model of secondary hypertension, also produced marked hypotensive effects in the blood pressure, on the order of about 15 mm Hg to 20 mm Hg in both the systolic and diastolic blood pressures.

The experiments as set forth in Examples 8 through 10 demonstrate that an antibody to the protein associated with hypertension will produce a significant reduction in blood pressure. This indicates that the protein associated with hypertension has a significant hypertensive effect. Thus, these experiments indicate that blocking the function of the protein associated with hypertension causes a significant hypotensive effect. These experiments demonstrate one type of agent capable of blocking the hypertensive effect of the protein associated with hypertension, namely antibody to the protein associated with hypertension. However, other agents that block the function of the protein associated with hypertension will also produce a reduction in blood pressure, and are considered part of the present invention.

One such other agent is an agent capable of selectively binding to the protein associated with hypertension. The selectively binding agent may be either a competitive or a noncompetitive selective binding agent. It is not necessary that the selective binding agent be an antibody to the protein associated with hypertension. Examples are such agents as specific affinity resins, analogs of a receptor for the protein associated with hypertension, etc.

Another example of an agent which blocks the function of the protein associated with hypertension may be an agent which inhibits the interactions of the protein associated with hypertension which are responsible for the hypertensive effect. The inhibiting agent may be competitive or noncompetitive. Examples include analogs of the protein associated with hypertension, peptides of the protein associated with hypertension, analogs of the peptides of the protein associated with hypertension, agents which block the availability of receptors for the protein associated with hypertension, etc.

Another class of agents which block the function of the protein associated with hypertension includes agents capable of modifying the protein associated with hypertension to block the hypertensive effect of the protein associated with hypertension, where the modifying agents may be chemical or enzymatic modifying agents. Thus, specific enzymes may cleave the protein associated with hypertension so that it will be degraded to a point where it no longer has a hypertensive effect. Chemical means for doing substantially the same thing may also be developed.

A second series of experiments was done in which antibody to the protein associated with essential hypertension in humans was induced in SHR rats to study the cross-reactivity and function of the protein associated with essential hypertension in humans.

### Example 11

The isolation of the protein associated with essential hypertension in humans was carried out in the same way as set forth in Example 8 with respect to the isolation of the protein associated with hypertension from the SHR rats. The only difference was that a human plasma sample known to contain the protein associated with essential hypertension (having a relative molecular weight of about 14,000) was used. Gel slices containing the protein associated with essential hypertension were homogenized by being passed through syringe needles from 18 gauge to 25 gauge and were soaked in phosphate buffer overnight.

The homogenate was mixed with an equal volume of Freund's adjuvant and injected subcutaneously in an SHR rat (about 0.5 ml/rat). A litter mate of the SHR rat was injected with the adjuvant mixed with buffer for a sham immunization. The rats were about 20 weeks old and weighed about 250 g to 300 g when injected.

Two weeks after the injections, a cannula was implanted in the left carotid artery of the injected SHR rats for blood pressure measurement.

The results obtained are set forth in the following table:

## Table 4

| Animal | Type of Immunization | Post Immunization Blood Pressure (mm Hg) |
|---|---|---|
| SHR | Sham Immunized | 210/148 |
| SHR | Actively Immunized | 140/105, Five days later 156/120 |

The blood pressure for the SHR rat actively immunized with the protein associated with human essential hypertension was tremendously lower than the blood pressure of the sham immunized litter mate. The blood pressure remained at a significantly reduced level for several days after implantation of the cannula. The protein associated with human essential hypertension that was injected into the SHR rat acted as an antigen which induced antibody to the protein associated with hypertension in the actively immunized SHR rat.

To verify that antibody to the protein associated with human essential hypertension was elicited in the actively immunized SHR of Example 11, a further experiment was done. The experiment was set forth in the following specific, non-limiting example.

### Example 12

Serum was obtained from the actively immunized SHR rat described in Example 11. One-half of each of several capillary tubes contained the serum diluted 1:1 with phosphate buffered physiological saline solution. The other half of each tube contained a sample of human plasma diluted 1:1 with phosphate buffered physiological saline solution.

The human plasma samples were taken from patients with essential hypertension and were known to contain a protein associated with human essential hypertension. The tubes were left undisturbed for a couple of days in a refrigerator. Upon inspection, it was discovered that precipitates comprising antigen-antibody complexes had formed in the tubes. By preparing analytical electrophoretic gels in accordance with the method set forth in Example 5, it was verified that the immune precipitate contained the protein associated with human essential hypertension.

This means that the actively immunized SHR rat in Example 11 actually produced antibody to the protein associated with human essential hypertension. This experiment also verifies an additional method of detecting the existence of a protein associated with hypertension, and more specifically, a protein associated with human essential hypertension.

In basic terms, the antibody-based detection method comprises the steps of:

(a) inducing antibody to the protein associated with hypretension;

(b) obtaining a sample of a solution containing the antibody;

(c) mixing the solution containing the antibody with a body fluid being tested for the protein associated with hypertension; and

(d) detecting the existence of an immune complex in the mixture which indicates the presence of the protein associated with hypertension in the body fluid being tested.

The present invention may be embodied in other specific forms without departing from the spirit or essential attributes thereof and, accordingly, reference should be made to the appended claims, rather than to the foregoing specification, as indicating the scope of the invention.

CLAIMS:

1. A composition for treating mammalian hypertension evidenced by the presence in a body fluid of the mammal of a protein associated with hypertension, characterized by
comprising an agent which blocks the hypertensive effect of the protein and contains an antibody to the protein.

2. A composition according to claim 1, wherein the antibody has been made by:

(a) immunizing a mammal with the protein associated with hypertension;

(b) removing blood from the mammal after a time sufficient for antibody to the protein to be present in the blood; and

(c) separating the antibody from the blood.

3. A composition according to claim 1 or 2, wherein the mammal is a human, the hypertension is essential hypertension and the protein is associated with human essential hypertension.

4. A composition according to claim 3, wherein the antibody has been made by:

(a) substantially isolating a protein associated with hypertension in a body fluid of a human having or being predisposed to essential hypertension;

(b) immunizing a mammal with the protein;

(c) removing blood from the mammal after a time sufficient for antibody to the protein to have formed in the blood; and

(d) separating the antibody from the blood.

5. A composition for treating mammalian hypertension,
characterized by
comprising an antigen which is a protein associated with hypertension in a mammal.

6. A composition according to claim 5, wherein the mammal is a human and the mammalian hypertension is essential hypertension.

7. A process for treating a mammal which has or is predisposed to hypertension, as evidenced by the presence in a body fluid of the mammal of a protein associated with hypertension,

characterized in

that a dose of an agent effective to block the hypertensive effect of the protein is administered to the mammal, the agent containing an antibody to the protein.

8. A process according to claim 7, wherein the mammal is a human, the hypertension is essential hypertension and the protein is associated with human essential hypertension.

9. A process according to claim 8, wherein the agent contains antibody made by:

(a) substantially isolating a protein associated with hypertension from a body fluid of a human having or being predisposed to essential hypertension;

(b) immunizing a mammal with the protein;

(c) removing blood from the mammal after a time sufficient for antibody to the protein to have formed in the blood; and

(d) separating the antibody from the blood.

10. A process according to claim 7, 8 or 9, wherein the agent is administered intra-arterially.

11. A process according to claim 7, 8 or 9, wherein the agent is administered intravenously.

12. A process for treating a mammal which has or is predisposed to hypertension,

characterized in

that the mammal is immunized by injecting a dose of an agent effective to induce in the mammal antibody to a protein associated with hypertension in the mammal.

13. A process according to claim 12, wherein the agent is a protein associated with hypertension in a mammal.

14. A process according to claim 12 or 13, wherein the hypertension is essential hypertension and the protein is associated with human essential hypertension.

FIG.1

0070167

# FIG. 2

—

6

5

—— 25.7K

4

—— 17.5K

3

—— 14.3K

P1

1

2

A        B        +

FIG. 3

# FIG.4

—

—68K

—43K

—29K

—21K

U1

9

—14.3K

7

10

8

+

C  D

FIG.5

**FIG.6**

Labels on gel: P5, P4, P2, P3

Molecular weight markers: 60K, 25.7K, 17.5K, 14.3K, 5K

Lanes: G  H  I  J  +

FIG. 7

FIG. 8

FIG.9

FIG.10

FIG.11

FIG.12